**Europäisches Patentamt**

**European Patent Office**

**Office européen des brevets**

(19)

(11) Publication number: **0 075 540**
**B1**

(12) # EUROPEAN PATENT SPECIFICATION

(45) Date of publication of patent specification: **22.10.86**

(21) Application number: **82810388.7**

(22) Date of filing: **17.09.82**

(51) Int. Cl.⁴: **C 08 L 33/02,** C 08 L 71/02,
C 09 J 3/14, A 61 M 31/00,
A 61 M 37/00, A 01 C 1/06 //
A61K47/00, A01N25/10

(54) **A coated absorbant substrate or shaped article, containing a water-soluble and/or volatile active agent.**

(30) Priority: **23.09.81 US 304752**

(43) Date of publication of application:
**30.03.83 Bulletin 83/13**

(45) Publication of the grant of the patent:
**22.10.86 Bulletin 86/43**

(84) Designated Contracting States:
**CH DE FR GB IT LI NL**

(56) References cited:
**GB-A-2 026 517**
**JP-A-75 033 976**
**US-A-3 387 061**
**US-A-3 968 310**
**US-A-4 249 531**

(73) Proprietor: **CIBA-GEIGY AG**
**Klybeckstrasse 141**
**CH-4002 Basel (CH)**

(72) Inventor: **Lee, Ping I., Dr.**
**6 Five Oaks Lane**
**Valley Cottage New York 10989 (US)**

Courier Press, Leamington Spa, England.

# 0 075 540

**Description**

The present invention relates to substrates of membrane-forming polymeric systems. Polymeric articles for the continuous dispensing of active agents into the environment of use are known. US—A—3,567,118 discloses fabricated articles coated with a crosslinked hydrophilic polymer containing a volatile active agent. As a result of the cross-linking and thermosetting nature of the polymer, the coating has to be polymerized in situ under elevated temperatures in the presence of the volatile ingredient. One immediate shortcoming of this approach is that the high temperature experienced during such processing invariably results in a change of chemical properties of the volatile active agent, apart from any premature evaporation loss. In addition, the small solubility of volatile active agents in the polymer often limits the level of incorporated actives thereby restricting the duration of release from such articles. Similar drawbacks exist in systems disclosed in US—A—3,705,938, i.e. non-porous polymer laminates, and in US—A—3,975,350, i.e. crosslinked polyurethane hydrogels.

US—A—3,578,545 and US—A—3,985,298 utilize microporous polymeric materials for preparing impregnated and laminated plastic sheets containing volatile ingredients. However, the microporous sheet in these constructions must have sufficient porosity to permit a particular volatile active agent to pass through. Furthermore, the construction of laminates described in the prior art frequently require cementing between polymer and substrate of dissimilar nature thereby increasing the processing difficulty and the frequency of delamination. Moreover, these prior art systems do not substantially minimize the undesirable fractionation among the components of a blended volatile active agent such as essential oils and pheromones. Fractionation is known to be undesirable since it reduces the efficacy and changes the quality of the volatile active ingredient. Thus, the pleasant fragrance that may initially be present will vary and disappear with the passage of time and with the resultant change in concentration of the various essential oil components. Correspondingly, the effective odor counteraction that may be achieved initially will also vary and diminish with time. Likewise, fractionation in pheromones may tend to impair the constancy of activity.

With respect to the disclosed polymer systems, the prior art describes either a crosslinked hydrophilic polymer system, which has to be thermoset in situ, or a hydrophobic thermoplastic system which has to be hot melt extruded. None of the polymers described in the above prior art exhibit easily adjustable permeability and hydrophilicity/lipophilicity balance to accommodate the release of active agents of different nature, hence unsatisfactory results are often obtained for the uses recited herein.

Further with regard to prior art systems, US—A—3,387,061 discloses the chemical reaction product of a polycarboxylic acid and a polymeric polyether having an average molecular weight in excess of 4,000. This reference indicates that polyethers of the indicated molecular weight are essential in order to form a precipiate and, subsequently, to form coatings. It is to be noted, however, that such high molecular weight systems have limited permeability, reduced stability and reduced solubility in water and lower alcohols. Solubilization of active ingredient in such systems is frequently limited. Accordingly, the ability for such polymer systems to effectively control the rate and duration of release of active ingredients is negligible. It should also be noted that in addition to the high molecular weight limitation, the lack of surfactant nature in the components severely limits the achievable loading level and permeability of diverse active agents when such prior art reaction products are used in conjunction with the systems contemplated in the present invention.

Furthermore, Japanese Patent Publication 75/33,976 discloses the separation of nonionic detergents from dilute aqueous solutions by the addition of an aqueous solution of polycarboxylic acid. However, an excess of polycarboxylic acid solution and the presence of a water soluble salt with a polyvalent cation are required for the separation process. Moreover, the incomplete removal of nonionic detergent and the resultant presence of various impurities in the precipitate creates uncertainty as to the exact composition of the precipitate, a clearly disadvantageous situation.

Accordingly, it is the primary object of the invention to provide a polymeric system, comprising an association product which is flexible and thermoplastic and which exhibits a wide range of permeabilities for varying active agents (ingredients) so as to be useful as membranes, coatings and carriers of various geometries, primarily for the controlled release of volatile and water-soluble active agents.

Another object of the invention is to provide a polymer system for the release of active agents at a controlled and uniform rate over a prolonged period of time, which system is relatively simple and economical to prepare.

Still a further object of the invention is to provide a polymer system which allows very high levels of active agent to be incorporated such that a more compact delivery system in the form of membranes, coatings and carriers of various geometries can be constructed.

Yet another object of the invention is to provide a delivery system which substantially minimizes fractionation among the components of mixed active agents such as essential oils and pheromones.

Still another object of the invention is to provide a polymer system which functions both as an adhesive layer as well as a rate controlling membrane for the release of active agents, when it is coated on or laminated to a substrate containing said active agents.

The foregoing objects of the invention are attained by the unexpected result that a blend of a polymeric carboxylic acid and an ethoxylated nonionic surfactant preferably at a weight ratio ranging at least from

2

1:20 to 20:1, or, more preferably, from 1:4 to 4:1, yields a rubbery and thermoplastic system having a low and adjustable glass transition temperature, and exhibiting flexibility in terms of permeabilities for various active agents. The polymeric carboxylic acids employed herein include those acidic polymeric materials in which the acidity is due to free carboxylic acid groups with a minimum of about 10% of the monomer units comprising carboxylic groups. The ethoxylated nonionic surfactants suitable for the purpose of the invention contain a minimum of 2 ethylene oxide units, have HLB (hydrophile-lipophile balance) values between 2 and 40 and have molecular weights ranging up to about 4,000. These compatible blends, which can be readily prepared at room temperature, are totally amorphous and distinctly different from the parent components with respect to their physical state, flexibility, glass transition temperatures, and permeabilities to a diversity of volatile and water-soluble active agents. The amorphous structure and the low glass transition temperature make it an ideal membrane material for the controlled release of active ingredients.

The used polymer system is unique over those of the prior art in as much as an adjustment in the ratio and type of polymeric carboxylic acid and ethoxylated nonionic surfactant components permits the practitioner to prepare polymers with extremely wide ranges of application possibilities, particularly with regard to permeabilities to various volatile and watersoluble active agents. The polymer systems are therefore particularly suitable as membranes, coatings, granules and other type of carriers for the controlled release of active agents over a prolonged period of time via a diffusion mechanism. Depending upon the particular environment in which the polymer system is used, applicable volatile active agents may be essential oil, medicament, pheromone, anti-microbial, antibacterial, anti-fungal, insecticidal, herbicidal, and other volatiles. The polymer system is equally applicable as a control release membrane for active ingredient which exhibit a minimum water solubility. The exact degree of water solubility will vary widely depending on the release requirements. Accordingly, for purposes of this invention, the term "active agent" is intended to include both volatile and water-soluble materials. The polymers are also applicable as protective coatings for a wide variety of substrates, as adhesives, and the like.

The incorporation of an ethoxylated nonionic surfactant is particularly advantageous in that the solubilization power of the surfactant enables a higher than usual level of active agent to be loaded into the polymer when it is used directly as an active agent carrier. Up to 50% or more loading can easily be achieved by first solubilizing the active agent in a nonionic surfactant in liquid state prior to the blending with polymeric carboxylic acid. This capability avoids the drawback of low active loading levels generally associated with the prior art systems.

Another advantage of the invention is that the polymer system and controlled release articles containing active agents made therefrom can readily be formed at room temperature, about 20—25°C, via mixing, coating or extrusion processes known to those skilled in the art. This capability obviates the decomposition and/or premature volatization of volatile active agents experienced in the prior art when processing under elevated temperatures. Moreover, when the polymer is coated on or laminated to a substrate containing an active agent, it uniquely serves a dual function as an adhesive and as a rate controlling membrane for the release of active agent into the intended environment. A further important advantage of the instant system is the ability to minimize undesirable fractionation among the components of a mixture type of volatile active agent such as essential oils and pheromones. As a result, there is a uniformity in composition and potency of the volatile active agent throughout the entire period of activity of the controlled release system.

Accordingly, the present invention relates to a coated absorbant substrate coated on one or both surfaces thereof with a polymeric system comprising an association product of a polymeric carboxylic acid having free carboxyl groups on at least 10% of the monomer units thereof, and an ethoxylated nonionic surfactant having at least 2 ethylene oxide units and a maximum molecular weight of about 4000, said absorbant substrate being impregnated with a volatile and/or water-soluble active agent, which are released at a controlled and uniform rate.

The present invention further relates to a shaped article prepared from a polymeric system comprising an association product of a polymeric carboxylic acid having free carboxyl groups on at least 10% of the monomer units thereof, and an ethoxylated nonionic surfactant having at least 2 ethylene oxide units and a maximum molecular weight of about 4000, which contains a volatile or water-soluble active agent therein, said active agent being released at a controlled and uniform rate.

The present invention further relates to a solid, water-soluble active agent coated with a polymeric system comprising an association product of a polymeric carboxylic acid having free carboxyl groups on at least 10% of the monomer units thereof, and an ethoxylated nonionic surfactant having at least 2 ethylene oxide units and a maximum molecular weight of about 4000 said water-soluble active agent being released at a controlled and uniform rate.

The present invention further relates to a sealed pouch prepared from the coated absorbant substrate according to the present invention. The present invention further relates to a seed coated with a polymeric system comprising an association product of a polymeric carboxylic acid having free carboxyl groups on at least 10% of the monomer units thereof, and an ethoxylated nonionic surfactant having at least 2 ethylene oxide units and a maximum molecular weight of about 4000.

The polymeric carboxylic acid which may be employed according to the present invention may generally be described as an acidic polymeric material in which the acidity is provided by free carboxyl groups. Such polymeric materials and methods for their preparation are well known in the art. Included among such acidic materials are synthetic polymers as well as natural polymers such as alginic acid, pectic acid and cellulose glycolic acid. An illustrative but by no means exhaustive listing of suitable polymeric carboxylic acid components include homopolymers of unsaturated carboxylic acids such as acrylic acid, methacrylic acid, and the like; copolymers of monocarboxylic acids of the acrylic series with one or more polymerizable vinyl or vinylidene compounds such as vinyl halides, vinyl acetate, vinyl benzoate, acrylonitrile, methacrolein, styrene, vinyl toluene, methyl methacrylate, ethyl acrylate, vinyl methyl ketone, vinyl methyl ether, t-butylacrylamide, N-dimethylacrylamide, and the like; or hydrolized copolymers of alpha, beta-ethylenically unsaturated dicarboxylic acid anhydrides, e.g. maleic anhydride with one or more terminally unsaturated mono-olefins such as ethylene, propylene, isobutylene, diisobutylene, chloroprene, and the like; or with cylic terpenes such as dipentene; or with vinyl or vinylidene compounds such as vinyl · halides, vinyl esters, vinyl ethers, vinyl ketones, styrene, acrylic acid and its esters, methacylic acid and its esters, and the like. Detailed descriptions of polymerizable vinyl, vinylidene and related compounds and the preparation of their copolymers known to the art are discussed in "Vinyl and Related Polymers" by Calvin E. Schildknecht, 1952, published by John Wiley & Sons, Inc.

It will be understood that the polymeric carboxylic acid which may be employed inventively may also be prepared by carboxyalkylation of polymers containing a multiplicity of hydroxyl groups such as polyvinyl alcohol, partially hydrolyzed polyvinyl acetate, cellulose and its derivatives, dextran and its derivatives, and the like; and by hydrolysis of polymers containing a multiplicity of acrylamide or acrylonitrile groups such as polyacrylamide, polyacrylonitrile, and the like in the presence of an alkaline catalyst. Carboxyalkylation of hydroxyl groups can be accomplished by methods well known to the art such as by reaction with chloroacetic acid in the presence of alkali, or by reactions with acid anhydrides derived from dicarboxylic acids such as phthalic anhydride, maleic anhydride, succinic anhydride, and the like to form half esters. Furthermore, the derivatives of any of the aforementioned polymers wherein in a fraction of the carboxylic acid groups are reacted to form derivatives thereof such as partial amid by treatment with ammonia and organic amines, and partial ester by treatment with lower alkyl alcohols may also be used. It is essential, however, that a minimum of 10% carboxylic acid groups be retained in the latter derivatives. In addition, the monomers and the resulting polycarboxylic acids according to the present invention may likewise be substituted by one or more other groups such as, halide, hydroxy, ester, ether, alkyl, aryl, phenoxy, alkylphenoxy, dialkylamino, perfluoroalkyl, perfluoroalkoxyperfluoroalkyl, polysilanyl-alkyl, thiol and the like. Other polymeric carboxylic acids according to the present invention may include a polycarboxylic acid ether having aliphatic chains alternating with and connected by ether oxygen to residues of a pentanoic acid described in US—A—3,300,444, and a carboxy polymethylene hydrocolloid based on the acrylic and methacrylic acid form of polymer containing 0.75 to 2% by weight of polyalkenyl polyether as a crosslinking agent as disclosed specifically in US—A—2,909,462. As previously noted, the overall composition of the polycarboxylic acid component is not critical, provided that at least 10% of the monomer units contain free carboxylic acid groups.

Preferred polymeric carboxylic acid components include polyacrylic acid, polyacrylic acid crosslinked with approximately 1% of polyallyl sucrose, polymethacrylic acid, polymaleic acid, polyitaconic acid, polyhydroxybenzoic acid, polygalacturonic acid, polyglutamic acid, polyglycollic acid, polylactic acid, ethylene/acrylic acid copolymer, ethylene/methacrylic acid copolymer, methylmethacrylate/methacrylic acid copolymer, methyl acrylate/methyl methacrylate/methacrylic acid terpolymer, ethyl acrylate/t-butyl acrylamid/acrylic acid terpolymer, methyl vinyl ether/maleic acid copolymer, vinyl acetate/crotonic acid copolymer, butadiene/maleic acid copolymer, polymaleic anhydride, acrylonitrile/maleic anhydride copolymer, butadiene/maleic anhydride copolymer, ethylene/maleic anhydride copolymer, 1-hexene/ maleic anhydride copolymer, 1-octadecene/maleic anhydride copolymer, methyl vinyl ether/maleic anhydride copolymer, n-octadecyl vinyl ether/maleic anhydride copolymer, styrene/maleic anhydride copolymer, vinyl acetate/maleic anhydride copolymer, cellulose, and carboxyl modified polyacrylamide.

More preferably, the polymeric carboxylic acid components include polyacrylic acid, an ethylene-maleic acid copolymer, styrene-maleic anhydride copolymer, 1-hexene-maleic anhydride copolymer, ethylacrylate-t-butyl acrylamide-acrylic acid terpolymer, polymethacrylic acid or cellulose acetate phthalate.

Within the preferred embodiment of this invention the polymeric carboxylic acid should have a minimum of 10%, and preferably above 20%, and still more preferably above 50% of the monomer units comprising carboxylic acid groups. In general, the average molecular weight of the polymeric carboxylic acid to be employed may range from about 1,500 up to 4,000,000 and higher.

The ethoxylated nonionic surfactants contemplated for use in the systems of this invention include ethoxylated alkylphenols, ethoxylated mono- and poly-hydroxy aliphatic alcohols, ethoxylated fatty amines, ethoxylated fatty acid amides and ethanolamides, ethoxylated fatty acids, ethoxylated fatty acid esters, ethoxylated sorbitan fatty acid esters, ethoxylated sorbitol esters, ethoxylated vegetable oils, ethoxylated lanolin derivatives, ethoxylated sugar derivatives, ethoxylated naphthalene derivatves, ethoxylated mercaptans, ethoxylated polypropylene glycols, ethoxylated fatty glycerides, ethoxylated fatty glycol esters, ethoxylated sucroglycerides. The applicable ethoxylated nonionic surfactants may be primary, secondary, tertiary, saturated, unsaturated, linear or branched in structure. The backbone and pendant chain of the said surfactants may be substituted in part by one or more other groups such as

4

halide, hydroxyl, ester, ether, alkyl, aryl, phenoxy, alkylphenoxy, dialkylamino, perfluoroalkyl, perfluoroalkoxyalkyl, silicone, silane, thiol and the like, e.g. the compositions disclosed in US—A—2,915,554 and US—A—4,171,282. Other ethoxylated nonionic surfactants applicable herein include perfluoroalkyl polyurethane surfactants as disclosed in US—A—4,046,944. The foregoing illustrative listing of applicable ethoxylated nonionic surfactants is by no means exhaustive of the surfactants which can be used, it being required of applicable surfactants that the ethylene oxide content be sufficiently large to enable them to behave in the manner of a nonionic surfactant. Detailed descriptions of ethoxylated nonionic surfactants and processes for their preparation known to the art are discussed in "Nonionic Surfactants", edited by M. J. Schick, 1970, published by Marcel Dekker, Inc., and in "Surfactants and Interfacial Phenomena" by M. J. Rosen, 1978, published by John Wiley & Sons, Inc., such teachings being incorporated by reference herein.

Preferred surfactants include octylphenoxy polyethoxy ethanols, nonylphenoxy-polyethoxy ethanols, ethoxylated sorbitan monolaurates, ethoxylated sorbitan monopalmitates, ethoxylated sorbitan monostearates, ethoxylated sorbitan monooleates, ethoxylated sorbitan tristearates, ethoxylated sorbitan trioleates, ethoxylated oleyl amides, ethoxylated tallow amides, ethoxylated glycol laurates, ethoxylated glycol stearates, ethoxylated glycol oleates, ethoxylated stearic acid, ethoxylated oleic acid, ethoxylated rosin fatty acids, ethoxylated lauryl ethers, ethoxylated cetyl ethers, ethoxylated stearyl ethers, ethoxylated oleyl ethers, ethoxylated tridecyl ethers, ethoxylated polydimethylsiloxanes, ethoxylated polypropylene glycols, ethoxylated polyurethanes, and ethoxylated perfluoroalkyl polyurethanes.

Within the preferred embodiment of this invention, the ethoxylated nonionic surfactants contemplated should comprise a minimum of 2 and preferably at least 5 ethylene oxide units and have HLB (hydrophilelipophile balance) values between 2 and 40 and preferably between 4 and 18. The HLB system identifies the lipophilic and hydrophilic character of surfactants and is fully described in a bulletin distributed by ICI Americas Inc. Surfactants with HLB values less than 10 are generally insoluble in water, while those with HLB values greater than 10 are generally water-soluble. In general, the average molecular weight of the ethoxylated nonionic surfactant to be employed may range from about 150 to 4,000 and preferably up to 3,500.

The process for the preparation of homogeneous blend (polymeric system) of the inventively used polymeric carboxylic acids and surfactants involves blending the indicated polymeric carboxylic acid and ethoxylated nonionic surfactant under conditions and subsequent treatment as explained hereinafter as determined by the end use requirements of the systems. Thus, since most of the blends so formed are relatively insoluble in water, it is preferred to dissolve the polymeric carboxylic acid and ethoxylated nonionic surfactant in separate solvents or water-solvent mixtures (which can be the same solvent or different but miscible solvents) in the desired concentration, and subsequently to add one solution to the other such that a solution of the blend is formed from which useful membranes, coatings and other finished forms such as carriers for the controlled release of active agents can be prepared. The solution of the said blend can also be prepared by dispersing the polymeric carboxylic acid in a nonsolvent or solvent-nonsolvent mixture, followed by mixing the resulting dispersion with a miscible or soluble ethoxylated nonionic surfactant. Depending upon the nature of the components in the blend, the applicable solvents and nonsolvents contemplated include alcohols such as methanol, ethanol, isopropanol, and the like; alkylene glycols such as ethylene glycol, propylene glycol, and the like; monoalkyl ethers of alkylene glycols such as monomethyl ether of ethylene glycol, and the like; aliphatic and aromatic hydrocarbons such as hexane, toluene, and the like; halogen substituted aliphatic and aromatic hydrocarbons such as methylene chloride, carbon tetrachloride, chlorobenzene, and the like; ketones such as acetone, methyl ethyl ketone, and the like; esters such as ethyl acetate, butylacetate, and the like; and water, dioxane, formamide, dimethyl formamide, dimethyl sulfoxide, and the like. When a water-solvent or solvent-nonsolvent mixture is involved, the amount of solvent employed should be at least sufficient to prevent precipitation of the blend. Generally, at least about 20% based on the total mixture weight, of solvent is required.

Within the preferred embodiment of this invention the homogeneous blend of a polymeric carboxylic acid, with a minimum of 10%, and preferably above 20%, and still more preferably 50% of the monomer units comprising carboxylic groups, and a nonionic surfactant, with a minimum of 2 and preferably at least 5 ethylene oxide units and with HLB values between 2 and 40 preferably between 4 and 18, should have a component weight ratio ranging at least from 1:20 to 20:1 and preferably from 1:4 to 4:1 and most preferably an optimum range from 2:3 to 3:2. The optimum solids content in the solution of said blend is readily determined by routine experimentation according to the viscosity requirements in the intended fabrication process, such as coating or extrusion. The temperature at which the polymer blend is formed can range from 10°C to 130°C and is preferably at an ambient or room temperature of about 20—25°C. The blend so formed, after evaporating the solvent by air-drying or under reduced pressure, is generally homogeneous, totally amorphous, thermoplastic, rubbery, and distinctly different from the parent components with respect to physical state, flexibility, glass transition temperature, and permeability to active agents. In every instance, the homogeneous blend of a brittle, solid polymeric carboxylic acid and a liquid or paste ethoxylated nonionic surfactant results in a rubbery, thermoplastic polymeric having a low and adjustable glass transition temperature which exhibits adjustable permeabilities for various active agents. The extent of change in properties of the blend from those of the starting components cannot be accounted for from simple mixing alone. Without wishing to be bound by a theory of operation, the probable mechanism by which the variation in property is achieved is through an intermolecular complex formation between the polymeric carboxylic acid and the ethoxylated nonionic surfactant in the solid phase. This is manifested by the observation that when an ethoxylated nonionic surfactant is blended with the aqueous solution of the polymeric carboxylic acid, an immediate increase in viscosity is observed which eventually results in the gelation of the mixture.

The polymeric association product employed according to this invention is generally soluble in an alkaline metal hydroxide solution with a pH-value above about 4.5. However, upon drying the said solution by air or under reduced pressure, a phase separation between the polymeric carboxylic acid salt and the ethoxylated nonionic surfactant would result if the polymeric carboxylic acid is neutralized to the extent that less than about 10% of the monomer units comprise free carboxylic acid groups. Thus, the presence of free carboxylic acid groups is essential in the association of a polymeric carboxylic acid and an ethoxylated nonionic surfactant in the solid state. As previously noted, the overall composition of the polymeric carboxylic acid component is not critical to the formation of polymeric blends providing it contains a minimum of 10%, preferably above 20%, and still more preferably at least 50% of the monomer units as carboxylic acid groups.

The polymeric association product is not limited to blends of a single polymeric carboxylic acid and a single ethoxylated nonionic surfactant. In fact, one or more polymeric carboxylic acids and one or more ethoxylated nonionic surfactants within the preferred embodiment of this invention can be utilized to form the blend. In addition, other compatible additives such as dyestuffs, pigments, plasticizers, UV-stabilizers, inorganic fillers including amorphous silica, bentonite and the like, and water soluble synthetic and natural polymers including polyvinyl alcohol, methyl cellulose, carboxymethyl cellulose, sodium alginate, carrageenan, and the like can be used in forming the polymer system of the present invention to improve the appearance, stability, solvent resistance, and mechanical properties of the resulting (shaped) articles.

The present invention is unique over prior art systems in a view of its adaptability to adjustment of the ratio and type of polymeric carboxylic acid and ethoxylated nonionic surfactant components to prepare polymers with an extremely wide spectrum of performance characteristics such as permeability to various active agents. The blends provided in this invention are therefore particularly suitable as membranes, coatings, granules and other type of carriers, with particular emphasis on the controlled release of volatile and water-soluble active agents into the environment of use over a prolonged period of time. The mechanisms of controlled release involves the dissolution of the active agent in the polymer phase, diffusion through the polymer and the desorption of ingredient from the external polymer surface into the environment of use.

The expression "volatile active agent" as used herein broadly includes any compound, composition of matter or mixture thereof with measurable vapor pressure at the temperature of intended usage, that can be delivered from the system of instant invention. Depending upon the particular environment of use in which the system of instant invention is used, the applicable volatile and/or water soluble active agents may include essential oils, pheromones, medicaments, germicides, algicides, biocides, fungicides, disinfectants, insecticides, pesticides, herbicides, and other volatiles. The term "essential oils" includes perfumes and other conventional volatile odorous air-treating agents. The term "pheromones" generally includes insect sex attractants and repellents. The term "medicaments" includes physiologically or pharmacologically active substances that produce a localized or systemic effect in humans and other animals. The active medicaments that can be delivered into the environment of use include bronchodilators, vasodilators, inhalational expectorants, and the like. The phrase "environment of use" as used herein refers to the atmosphere surrounding an active agent-releasing system including surfaces within its vicinity such as nasal receptors, respiratory tracts, skin, bark, soil, leaves, roots, insects, cytoplasmic membranes, and the like where the said volatile active agent can be deposited from vapor state or where said water-soluble active agent can be deposited in the liquid state.

The criteria for selecting an optimum component ratio of polymeric carboxylic acid and ethoxylated nonionic surfactant within the prescribed range, will vary according to the intended area of utility. However, a practitioner can readily ascertain the optimum ratio of components by testing the permeation and release properties of the resulting blend. This is achieved by following the weight loss of an active agent from an article prepared from the said blend as a function of time.

Another embodiment of the present invention is directed to the method of preparation of articles containing the blend described herein for the controlled and uniform release of active agents into the environment of use over a prolonged period of time. Thus, a viscous solution of the said blend can be coated directly onto an absorbent substrate by conventional means such as dip-coating, doctor blades, wire-wound rods, roller mills, gravure rolls, and the like, followed by air drying or drying under reduced pressure. The absorbent substrate in the form of sheet, pad, granule, cylinder or other convenient shape may either be a woven or nonwoven material including cloth, felt, paper and the like, comprising cellulosic fibers, cellulose ester fibers, crosslinked polyvinyl alcohol fibers, polyvinyl chlorid fibers, acrylonitrile fibers, glass fibers, nylon fibers, polyethylene terephthalate fibers, wools, silks, and the like. Alternatively, foamed polymeric material such as open pore polyurethane foam and the like can also be utilized.

The said absorbent substrate can then be impregnated with a volatile active agent either prior to or after the coating process but preferably the latter. Optionally, after the impregnation of the coated substrate with a volatile active agent, the uncoated side of the substrate can be coated with a viscous solution of the blend and laminated either to a barrier backing material impermeable to the said volatile active agent such as aluminium foil, high density polyethylene film, polyvinylidene chloride film, polyacrylonitrile film and the like, or to the uncoated side of similarly prepared impregnated substrates to form a sandwich structure. The articles so formed contain a volatile active agent reservoir enclosed with a release rate-controlling membrane and therefore provide a prolonged and controlled release of the said volatile active agent into the environment of use via a solution-diffusion mechanism.

With respect to the direct coating onto an absorbent substrate, the viscosity of the solution of the polymer blend described herein is generally increased by adjusting the solids content to an extent such that it is flowable and castable yet still viscous enough to prevent any appreciable penetration into the absorbent, porous substrate. Consequently, any adverse effect on the rate controlling property of the coated membrane layer is avoided. Alternatively, and preferably, a thin sheet of woven or nonwoven material of lesser porosity than the absorbent substrate but still freely permeable to various volatile active agents can be coated with a viscous solution of the blend described herein to form a supported, penetration free membrane. Before being completely dry, the said membrane coated sheet exhibits slight tack and thus can be laminated to the absorbent substrate described previously. The thin sheet of woven or nonwoven material employed herein can also be decorated or printed to provide an attractive appearance. Still another manner of fabricating the said coated article is by casting the coating solution of the blend onto a release liner such as silicone treated release paper, Teflon film of polytetrafluoroethylene (Teflon—Trademark) and the like to form a penetration free membrane. After evaporating the solvent by air-drying or under reduced pressure, the membrane is thermally or mechanically transferred from the release liner onto the previously described absorbent substrate under an applied pressure of, for example, $7 \times 10^{-2}$ to 3,5 bar (1 to 50 psi) at a temperature of 50 to 130°C.

An important advantage of the said membrane coated or laminated articles for the controlled release of volatile active agents is their unexpected property of minimizing fractionation among the components of a mixture type of volatile active agent such as essential oils and pheromones. Thus, contrary to prior art systems which exhibit fast release of the low boiling components followed by release of the components boiling at medium and high temperature ranges, the instant systems exhibit similar percentage releases for all three portions through the rate controlling membrane. As a result, there is uniformity in composition and potency of volatile active agent throughout the entire period of activity of the controlled release system.

Another method for the preparation of the blend according to the present invention and controlled release articles containing active agents made therefrom involves solubilizing an active agent directly in an ethoxylated nonionic surfactant in liquid state prior to the blending with a liquid or powdery polymeric carboxylic acid without any solvent. The incorporation of an ethoxylated nonionic surfactant as one of the components in the present invention is particularly advantageous in that the solubilization power of the surfactant enables a higher than normal level, up to 50% or more, of active agents to be incorporated into the polymer when it is used directly as an active agent carrier. This high level of active agent loading would have been thought to be impossible to achieve in prior art systems due to phase separation.

The active agent-containing blend so formed has the consistency of a plastic dough. By changing the nature and fraction of the ethoxylated nonionic surfactant in the blend or by dissolving the ethoxylated nonionic surfactant in a small amount of solvent prior to the formation of the blend, the plasticity of the blend can be adjusted to suit a desired fabrication process. Thus, the active agent-containing blend so prepared may be molded or extruded to form shaped articles, sheets, cylinders, granules and the like at ambient or room temperature, about 20 to 25°C. They may also be foamed or blown by techniques known to those skilled in the art for paste or organosol compositions and may further be mixed with inert fillers, dyes, pigments, and the like. Alternatively, the blend may be dissolved in a suitable solvent and then used as a coating for granulated materials, woven and nonwoven materials, paper, wood, metal and plastic surfaces, and surfaces of other shaped articles for the controlled release of incorporated active agents into the environment of use over a prolonged period of time.

The unique polymer systems are also suitable for use in a broad range of applications unrelated to the controlled release of volatile active ingredients. Thus, they can be used as coatings for solid, non-volatile active ingredients to provide controlled release thereof. For example, they can function as coatings for pharmaceutical tablets to provide controlled release in-vivo in humans and animals. They can provide comparable controlled release of herbicides and insecticides. They can be used strictly as protective coatings for a wide variety of substrates, providing both water and oil repellancy. In the latter area, they can function as bandage-type materials to protect skin abrasions and other wounds. The systems can be formed into pouches for release of a wide variety of materials, including delivery of medications to the skin or mucosa. As previously noted, incomplete drying of the films will provide a residual tackiness, enabling the systems to be utilized as adhesives with excellent tear and peel strengths. Finally, the systems can be utilized as seed coatings to provide greater control of the germination process without adversely effecting the total percentage of seed germination. Such coatings can also function to adjust the size of the seed where such adjustment is desirable. Correspondingly, fungicides, safeners, and the like, can be incorporated in the polymeric systems for controlled release during the entire germination cycle.

The following examples will further illustrate the embodiments of the present invention. In these examples, all parts and percentages given are by weight unless otherwise noted.

7

# 0 075 540

Examples 1—14 deal with the preparation of various polymer blends of the present invention and identification of their associated physical characteristics utilizing test methods such as X-ray diffraction, differential scanning calorimetry, tensile measurement, and like.

A) Manufacture of polymeric association products

Example 1

A blend is prepared utilizing 25 parts of octylphenoxy polyethoxy ethanol having 9 to 10 ethylene oxide units, an HLB value of 13.5 and an average molecular weight of 628, and 125 parts of 20% aqueous solution of polyacrylic acid having an average molecular weight of 250,000. An immediate increase in viscosity followed by precipitation is observed upon mixing. After air drying at room temperature, a clear, rubbery, thermoplastic polymer having a polyacrylic acid to octylphenoxy polyethoxy ethanol ratio of 1 to 1 suitable for use in the instant invention to provide prolonged and controlled release of volatile active agent is obtained. This polymer is determined to be totally amorphous having a low glass transition temperature Tg of −15.5°C and a tensile strength of 19,9 bar (283 psi) at 25°C.

Example 2

The procedure of Example 1 is repeated using 12.5 parts of said octylphenoxy polyethoxy ethanol and 187.5 parts of said 20% aqueous solution of polyacrylic acid. After air drying at room temperature, a clear, highly flexible, thermoplastic polymer having a polyacrylic acid to octylphenoxy polyethoxy ethanol ratio of 3 to 1 is obtained. This polymer is less flexible than the product of Example 1 and is determined to be totally amorphous having a glass transition temperature Tg of −2°C and a tensile strength of 39,5 bar (560 psi) at 25°C.

Example 3

The procedure of Example 1 is repeated using 37.5 parts of said octylphenoxy polyethoxy ethanol and 62.5 parts of said 20% aqueous solution of polyacrylic acid. After air drying at room temperature, a clear, rubbery, thermoplastic polymer having a polyacrylic acid to octylphenoxy polyethoxy ethanol ratio of 1 to 3 is obtained. This polymer is more flexible than the product of Example 1 and is determined to be totally amorphous having a glass transition temperature Tg of −35.5°C.

Example 4

A 20% aqueous solution of ethylene-maleic acid copolymer is prepared from an ethylene maleic anhydride resin with an average molecular weight of 100,000 (EMA-31, trademark). Thereafter, 25 parts of the solution are blended with 10 parts of 50% aqueous solution of a polyethylene glycol stearate ester with average molecular weight about 1,800 and HLB value of 17.3 (PEG 1540, trademark) to form a 1 to 1 blend. An immediate increase in viscosity is observed upon mixing. After drying overnight at 40°C, the product is clear, flexible and insoluble in water, lower alcohols and dimethylformamide (DMF) in contrast to the brittleness and good water, alcohol and DMF solubility of the above mentioned ethylene—maleic anhydride resin as shown in Table 1.

Example 5

Example 4 is repeated except that a monooleate (average molecular weight of 1,800 and HLB value of 17.0) is used instead said monostearate. After drying overnight at 40°C, the resulting product is also clear, flexible and insoluble in water, lower alcohols and DMF as shown in Table 1.

Example 6

Approximately 5 parts of a styrene-maleic anhydride resin having an average molecular weight of 50,000, is dissolved in 25 parts of acetone to form a solution with a solids content of 16.67%. The solution is then mixed with 10 parts of a 50% aqueous solution of the monooleate according to Example 5 to form a 1 to 1 blend. An immediate increase in viscosity is observed in mixing. After drying overnight at 40°C, the product is clear, moldable and insoluble in lower alcohols, acetone and DMF in comparison with the brittleness and good alcohol, acetone and DMF solubility of styrene maleic anhydride resin as shown in Table 1.

Example 7

Example 6 is repeated except that an ethoxylated fatty acid amide with average molecular weight of 2,478 and 50 ethylene oxide units, (Ethomid HT/60, trademark) is used instead of said monooleate. After drying overnight at 40°C, the resulting product is clear, flexible and insoluble in lower alcohols, acetone and DMF as shown in Table 1.

Example 8

Example 6 is repeated except that an ethoxylated sorbitan monooleate having 20 ethylene oxide units, an HLB value of 15 and an average molecular weight about 1,300 (Tween 80, trademark) is used instead of said monooleate. After drying overnight at 40°C, the resulting product is also clear, flexible and insoluble in lower alcohols, acetone and DMF as shown in Table 1.

8

## TABLE 1
### Solubility characteristics of association polymers at 25°C

| Description | Composition | Water | Ethanol | Methanol | Acetone | DMF | Description |
|---|---|---|---|---|---|---|---|
| Ethylene maleic anhydride copolymer (EMA) | 100% | S | S | S | I | S | y, b |
| EMA/PEG 1540 monostearate (Ex. 4) | 1:1 | I | I | I | I | I | c, f |
| EMA/PEG 1540 monooleate (Ex. 5) | 1:1 | I | I | I | I | I | c, f |
| Styrene maleic anhydride copolymer (SMA) | 100% | I | S | S | S | S | c, b |
| SMA/Ethomid HT/60 (Ex. 7) | 1:1 | I | I | I | I | I | y, t, f |
| SMA/PEG 1540 monooleate (Ex. 6) | 1:1 | I | I | I | I | I | c, m |
| SMA/Tween 80 (Ex. 8) | 1:1 | I | I | I | I | I | y, t, f |

Key    Solubility:  S=Soluble    Description:  b=brittle    m=moldable
                      I=Insoluble                      c=clear        t=transparent
                                                f=flexible      y=yellow

Example 9

Approximately 15 parts of cellulose acetate phthalate containing 19 to 23.5% acetyl groups and 30 to 36% phthalyl groups is dissolved in 85 parts of a mixture of 249 parts anhydrous acetone and 1 part of water to form a solution having a viscosity range of 0,05 to 0,09 Pa · s at 25°C. The solution is then blended with 22.5 parts of the ethoxylated sorbitan monooleate according to Example 8 and an immediate increase in viscosity was observed.

The solution so formed is knife cast onto a glass plate. After air drying at room temperature, a clear, flexible, thermoplastic and slightly elastic film having a polymeric carboxylic acid to ethoxylated nonionic surfactant ratio of 2 to 3 is obtained which is suitable for use in the instant invention to provide prolonged and controlled release of active agents.

Example 10

Approximatley 2 parts of a linear copolymer of 1-hexene and maleic anhydride with average molecular weight about 50,000 (PA-6, trademark) is dissolved in 18 parts of ethanol to form a solution of 10% solids content. Approximately 2 parts of this solution are mixed with 0.2 parts of a nonylphenoxy polyethoxy ethanol having 11 ethylene oxide units, HLB value of 13.8 and an average molecular weight of 704 (Triton N-111, trademark) to form a 1 to 1 blend. An immediate increase in viscosity is observed. After drying at 40°C for 2 hours, a clear, elastic film suitable for use in the instant invention to provide prolonged and controlled release of active agents is obtained.

Example 11

Example 10 is repeated except that the ethoxylated sorbitan monoolate according to Example 8 is substituted for the nonylphenoxy polyethoxy ethanol according to Example 10. After drying at 40°C for 2 hours, a clear, elastic film is formed.

Example 12

Five parts of 50% ethanol solution of a 40:40:20 mole % ethyl acrylate, t-butyl acrylamide and acrylic acid terpolymer having about 20% carboxylic acid content and an average molecular weight of 35,000 is mixed with 2.5 parts of a nonylphenoxy polyethoxy ethanol, having 9 to 10 ethylene oxide units, HLB value of 13.4 and an average molecular weight of 642 (Triton N-101, trademark) to form a 1 to 1 blend. An immediate increase in viscosity is noticed. Upon drying at 40°C for 2 hours, an elastic, clear film product is obtained.

Example 13

One part of the nonylphenoxy polyethoxy ethanol according to Example 12 is blended with 20 parts of a 2.5% aqueous solution of polymethacrylic acid having an average molecular weight of about 200,000. Immediate precipitation is observed upon mixing. After air drying at room temperature, a clear, rubbery, thermoplastic polymer having a polymethacrylic acid to surfactant ratio of 1 to 2 is obtained which is suitable for use in the instant invention to provide prolonged and controlled released of active agents.

Example 14

Example 13 is repeated except that the ethoxylated sorbitan monooleate according to Example 8 is used as a surfactant. Immediate precipitation is observed upon mixing. After air drying at room temperature, a clear, rubbery, thermoplastic polymer having a polymethacrylic acid to surfactant ratio of 1 to 2 is obtained which is suitable for use in the instant invention to provide prolonged and controlled release of active agents.

B) Application examples

Example 15

This example illustrates a typical method of preparation of controlled release articles using the polymer systems of the present invention as well as the advantages obtained from such systems.

25 parts of octylphenoxy polyethoxy ethanol having 9 to 10 ethylene oxide units, an HLB value of 13.5 and an average molecular weight of 628, are blended with 125 parts of 20% water/isopropanol (3 to 1 weight ratio) solution of polyacrylic acid having an average molecular weight of 250,000. The presence of isopropanol as a cosolvent prevents the precipitation of the blend. The resulting solution of the blend has a polyacrylic acid to octylphenoxy polyethoxy ethanol ratio of 1 to 1, a solids content of 33.3% and a Brookfield viscosity of 10 Pa · s (10,000 cps) at 25°C.

The solution so formed is knife coated with a 0,25 mm (10 mil) wet laydown onto one surface of a 1,125 mm (45 mil) blotter paper [95,25 kg (210 lb) basis weight]. Immediately after the casting, the coated blotter is air dried at 50°C. The uncoated side of the absorbent blotter is then impregnated with approximately 392,66 g/m² (0.08 lb/ft²) of citrus essential oil. Subsequently, this impregnated side is knife coated with the same viscous coating solution described hereinabove and laminated with the uncoated side of another identically impregnated blotter to form a sandwich structure. The finished product is then cut into desired shape and size for use in determining the controlled release of the said citrus essential oil.

The articles so formed exhibit a uniform and prolonged release of the citrus essential oil as evidenced by a $t_{50}$, time to reach 50% release, of 19 days and a total effective release period in excess of two months.

Control samples are prepared in the same manner as described in the preceding paragraphs except that the surface coatings using the identified polymer blend are omitted. The release of citrus essential oil from these articles is fast and uncontrolled with a $t_{50}$ of only 2 days and a total effective release period of only 3 weeks.

Example 16

This example illustrates a further advantage of the instant system in terms of reducing fractionation effects.

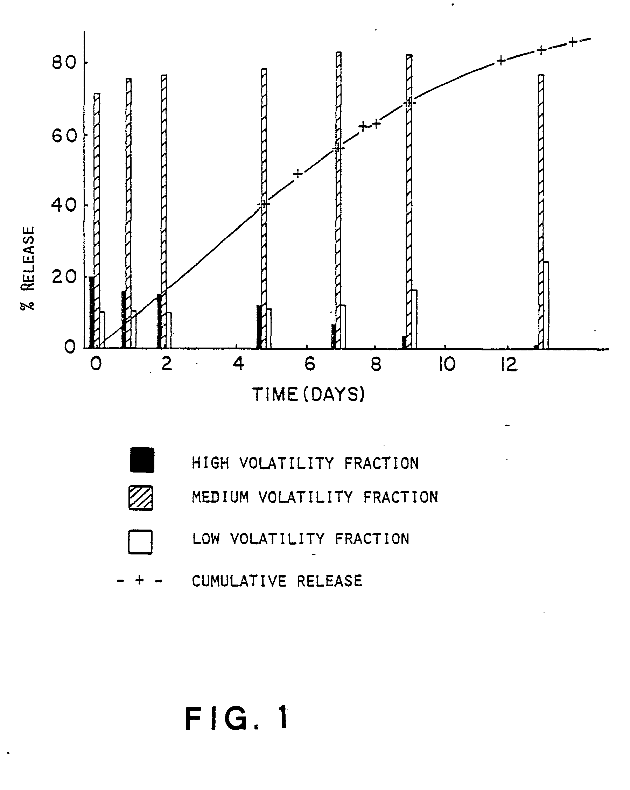

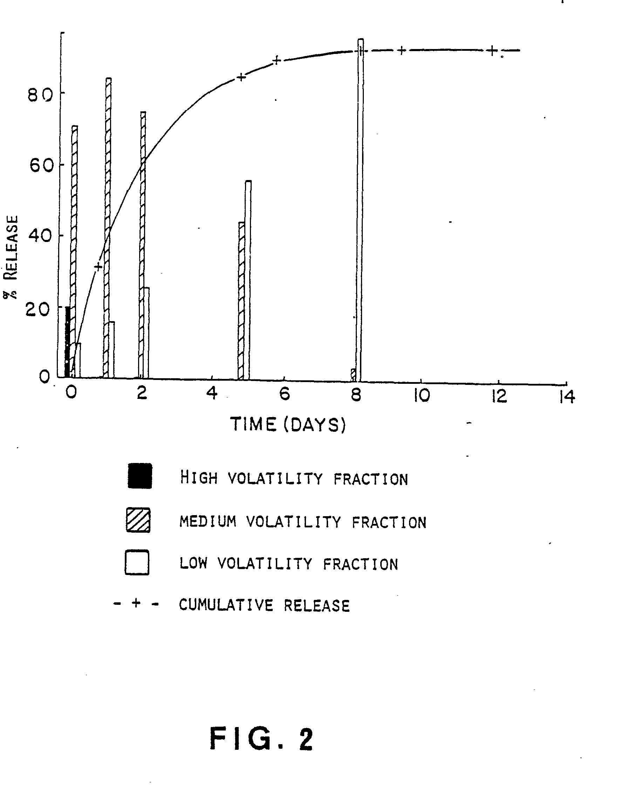

Initially, eight duplicate membrane laminated articles of identical dimensions are prepared in the same manner as in Example 15 except that about 490 g/m² (0.1 lb/ft²) citrus essential oil and a 0,125 mm (5 mil) wet laydown coating thickness are utilized with release from these articles being followed gravimetrically. Periodically, one article is removed from the test and the residual citrus oil extracted in methylene chloride overnight. The composition of the extract is determined by Gas-Liquid Chromatography (GLC). Comparable analyses are conducted on control articles without membrane coating prepared in the same manner as in Example 15. The citrus essential oil components are categorized as high volatility (0—15 minute retention time) and low volatility (23—31 minute retention time). The cumulative release and fractionation characteristics are presented in Figures 1 and 2.

More specifically, Figure 1 is a graphic depiction of the release of the essential oil, both cumulatively and in terms of its residual fractions, with the passage of time through the membrane laminated article, while Figure 2 is a similar graphic depiction of release versus time from an uncoated blotter.

The graphs described herein above thus clearly reveal the substantial reduction in essential oil fractionation and the uniform and prolonged release of active agent from the instant systems as compared to that of the uncoated control wherein fast, uncontrolled release and undesirable essential oil fractionation prevail.

Example 17

This example illustrates the adhesive nature of the polymer system of the instant invention and the unique advantage of said system in serving as a combined adhesive and rate controlling membrane.

A coating solution prepared in the same manner as in Example 4 is cast in a 7 mil wet laydown, using a knife coater, onto the surface of a 3 mil buisiness grade paper (Hyolite Sub 40, trademark) to form a paper supported membrane. The coated sheet is partially dried in a hot air oven at 80°C whereupon said membrane coated sheet exhibiting slight tack is laminated to the blotter paper stock previously used in Example 15. About 392,66 g/m² (0.08 lb/ft²) of herbal essential oil is then impregnated into said absorbent blotter from the unlaminated blotter side. Subsequently, this blotter side is coated with the same viscous coating solution described hereinabove and laminated with the uncoated side of another identical impregnated blotter to form a sandwich structure. The finished product is then cut into desired shape and size for use in determining the controlled release of said herbal essential oil. The articles so formed exhibit a uniform and prolonged release of herbal essential oil for a period of over two months.

Example 18

This example illustrates the heat activated adhesive property of the polymer system of the instant invention.

Example 17 is repeated except that the paper supported membrane is completely dried and laminated to the absorbent blotter using a heated roller. The article so formed exhibits a uniform and prolonged release of herbal essential oil for a period of over two months.

Example 19

This example illustrates the moisture activated adhesive property of the instant polymer system.

Example 17 is again repeated except that the paper supported membrane is completely dried and laminated to the absorbent blotter after wetting the coated surface. The articles so formed also exhibit a uniform and prolonged release of herbal essential oil for a period of over two months.

Example 20

This example demonstrates the thermal transfer of the polymer system of the present invention to a non-woven substrate.

A coating solution is prepared in the same manner as in Example 15, except that nonylphenoxy polyethoxy ethanol having 9—10 ethylene oxide units, an HLB value of 13.4 and an average molecular weight of 642 is substituted for the octylphenoxy polyethoxy ethanol. The resulting solution has a solids content of 33.3% and a Brookfield viscosity of 10 Pas at 25°C.

The solution so formed is knife coated onto the surface of a silicone release liner with 0,25 mm (10 mil) wet laydown. After drying in a hot air oven at 80°C, the membrane is transferred from the silicone release liner to the surface of a thin non-woven material (Reemay, style 2111, trademark) by being passed through heated squeeze rollers. This procedure yields a nonwoven, supported release rate controlling membrane

11

suitable for subsequent lamination to an appropriate substrate to provide prolonged and controlled release of volatile active agent.

Example 21

This example illustrates the heat sealability of the polymer system of the present invention and its versatility in device construction.

A coating solution prepared in the same manner as in Example 15 is knife coated with a 0,125 mm (5 mil) wet laydown onto the surface of a 0,075 mm (3 mil) buisiness grade paper (Hyolite Sub 40, trademark) to form a paper supported membrane. The paper supported membrane is completely dried in a hot air oven at 80°C and then formed into an open pouch by heat sealing three sides. An absorbent felt is inserted through the opening and 392,66 g/m² (0.08 lb/ft²) of citrus essential oil is impregnated into the felt. The pouch is subsequently heat sealed on the forth side. The article so formed exhibits a uniform and prolonged release of citrus essential oil with $t_{50}$ of 40 days and a total effective release period of three months.

Example 22

This example illustrates another manner of forming controlled release articles using the polymer system of the present invention.

One gram of citrus essential oil is first solubilized in 2 grams of nonylphenoxy polyethoxy ethanol having 15 ethylene oxide units, an HLB value of 15.0 and an average molecular weight of 880. Thereafter, the mixture is blended with 2 grams of powdered polyacrylic acid (average M.W. 250,000) in the absence of any solvent to form a plastic dough. Subsequently, it is compressed to form a flat sheet of area 20.97 cm² and thickness of 0.2 cm.

The articles so formed exhibited a uniform and prolonged release of citrus essential oil for a period of over two months.

Example 23

This example illustrates the controlled release of medicament in articles prepared from the polymer system of the present invention.

Thus, Example 17 is repeated except that eucalyptol, an inhalational expectorant, is used as the active agent instead of herbal essential oil. The articles so formed exhibit a uniform and prolonged release of eucalyptol for a period in excess of two months.

Control samples are prepared in the same manner except that the paper supported membrane is absent. The release of eucalyptol from these samples is fast and uncontrolled.

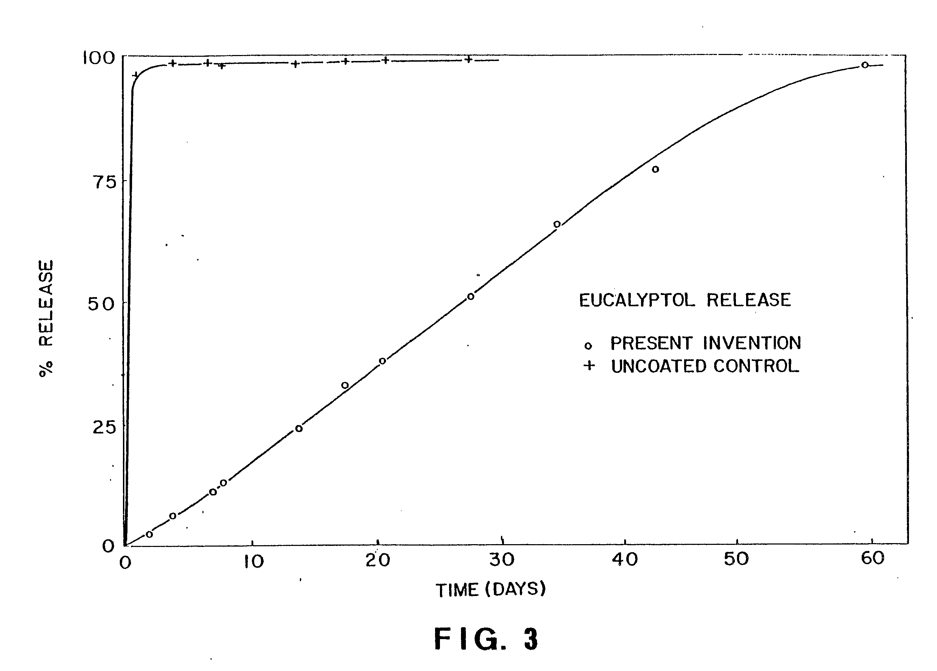

This pattern of release is depicted in Fig. 3. Thus, Fig. 3 is a graphic illustration of the % release of eucalyptol with the passage of time through the membrane laminated article as well as through an uncoated blotter.

Example 24

This example illustrates the controlled release of pheromones in articles prepared from the polymer system of the present invention.

25 parts of a nonionic surfactant based on ethoxylated polydimethylsiloxane having 16—20 ethylene oxide units and an average molecular weight of 1,200 is blended with 167 parts of a 15% ethanol solution of polyacrylic acid having an average molecular weight of 250,000 to form a viscous solution.

The procedure of Example 17 is then repeated using the above coating solution except that 5% of a boll weevil sex attractant (Grandlure, trademark) in mineral oil is used as the active agent instead of herbal essential oil. The articles so formed exhibit a uniform and prolonged release of the attractant for a period of over two months.

Example 25

This example illustrates the extrudable nature of the polymer system of the present invention.

4 parts of a polyacrylic acid of molecular weight about 200,000 to 300,000 highly crosslinked with about 1% of polyallyl sucrose, (Carbopol 934, trademark) is blended with 10 parts of the ethoxylated sorbitan monooleate according to Example 8 and 5 parts of isopropanol to form a highly plastic paste. The plastic paste so formed is extruded at room temperature to form cylindrical rod shaped articles.

Example 26

This example illustrates the utilization of the instant polymers in forming oil and water repellant film coatings.

0.5 parts of an ethoxylated perfluoroalkyl polyurethane surfactant (Lodyne T-21, trademark is blended with 10 parts of 5% aqueous solution of polyacrylic acid having an average molecular weight of 250,000. Immediate precipitation is observed. Two parts of ethanol are subsequently added to the precipitate to prepare a viscous solution. The solution so formed is dip-coated on a glass slide. After air drying at room temperature, the coating shows both water and oil repellency.

**0 075 540**

Example 27

This example illustrates the utility of the polymer system of the present invention as matrix material for the controlled release of pharmaceuticals.

About 250 parts of oxprenolol-hydrochloride, a β-blocker, is thoroughly mixed with 125 parts of the crosslinked polyacrylic acid according to Example 25 and 125 parts of the ethoxylated sorbitan monooleate according to Example 8. The mixture is subsequently compressed into a tablet on a laboratory tablet press.

The in-vitro release of the active ingredient from the tablet is followed by a UV spectrophotometric method. The tablet so formed exhibits uniform and prolonged release of active ingredient as evidenced by a $t_{50}$ of 2.5 hours and an effective release period of over 8 hours.

Example 28

Example 27 is repeated except a bronchodilator (Pseudoephedrin, trademark) is utilized therein. The tablet so formed exhibits uniform and prolonged in-vitro release of pseudoephedrine with $t_{50}$ of 2 hours and an effective release period of over 7 hours.

Example 29

This example illustrates the utility of the present polymer system in tablet coating.

About 0.5 parts of a diuretic (Theophylline, trademark) is blended with 2 parts of stearyl alcohol and then compressed into a tablet having a diameter of 0.93 cm and thickness of 0.55 cm. Another identical tablet is dip-coated with a coating solution consisting of a 1:1 weight blend of the polyacrylene acid and the monooleate used in Example 27 having a solids content of 7% in 1:1 ethanol/isopropanol mixture. After air drying of the coating, the coated tablet exhibits uniform and prolonged in-vitro release of the diuretic with a $t_{50}$ of 24 hours as compared with a $t_{50}$ of 21 hours of the uncoated control.

Example 30

This example illustrates the utility of the polymer systems of the present invention in preparing erodible devices for the controlled release of active agents.

Ten parts of an ethoxylated stearic acid having 10 ethylene oxide units and an average molecular weight of 718 (Ethofat 60/20, trademark) is blended with 66.6 parts of 15% ethanol solution of polyacrylic acid having an average molecular weight of 250,000 to form a viscous solution with a 26.1% solids content. Three-25 part portions of the solution are blended with 0.51, 1.33 and 4.47 parts, respectively, of the dimethylamine salt of 2,4-dichlorophenoxy acetate acid (2,4-D). These mixtures are then cast in molds to form sheets.

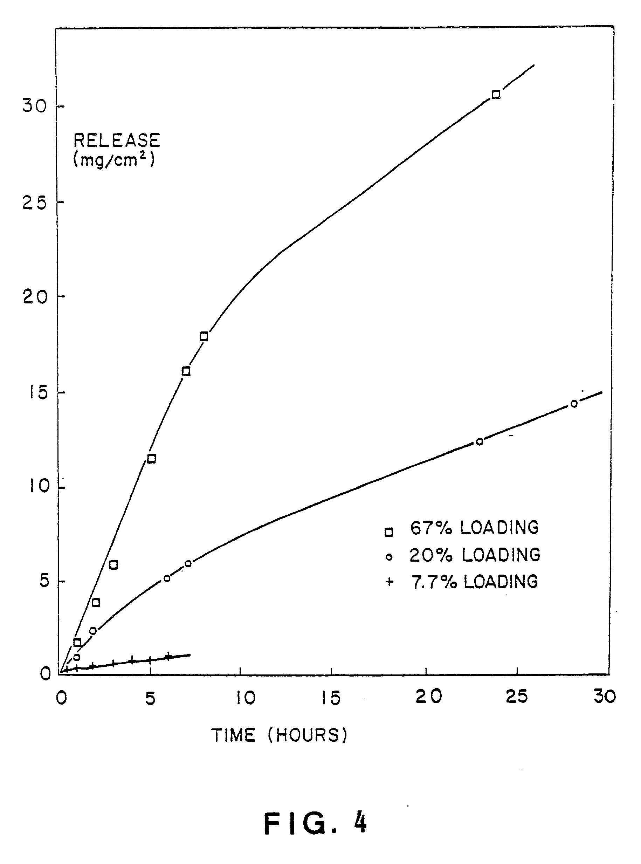

After drying under room temperature conditions, flat sheets (about 0.75 mm thick) with 7.7%, 20% and 67% of 2,4-D loading are obtained. About one square inch (6.45 cm$^2$) of each sheet is cut and adhered to an aluminium plate leaving the other surface exposed for the erosion test. The release of 2,4-D in aqueous media is measured by a UV spectrophotometric method. The articles so formed exhibit prolonged and controlled release of 2,4-D via a polymer erosion mechanism as shown in Figure 4. Thus, Figure 4 is a graphic depiction of the release of 2,4-D from the erodible sheet with the passage of time, showing the percent release for three different concentrations of herbicide.

Example 31

This example illustrates the utility of the polymer system of the present invention in the controlled delivery of systemically active pharmaceuticals to the skin or mucosa.

Thus, a 5 cm×5 cm pouch, containing 9.4 grams of a nitroglycerine in petrolatum ointment (Nitro-B, trademark), sandwiched between an impermeable aluminum foil backing and a paper supported membrane made in the same manner as in Example 17, is prepared by heat sealing along the edges with the membrane coated side facing the aluminum foil backing. The paper side of the supported membrane is then coated with a medical grade silicone adhesive for attaching the pouch to the target skin area.

The pouch so formed exhibits uniform and prolonged in-vitro nitroglycerine release for a period over 30 hours with a release rate around 20 g/cm$^2 \cdot$ hr as measured by liquid chromatography.

Example 32

Example 31 is repeated except that the paper supported membrane is cast from a blend of 4.76 parts of the polyacrylic acid according to Example 28, 4.76 parts of the monooleate used in Example 27 and 90.48 parts of 1/1 ethanol/isopropanol. The resulting dry membrane thickness (excluding the paper support) is about 0.025 mm. In addition, the ointment used in Example 31 is replaced with a 6.5% nitroglycerine-containing paste made from blending nitroglycerine on lactose (10% active) with mineral oil.

The article so prepared exhibits uniform and prolonged in-vitro nitroglycerine release for a period of over 30 hours with a release rate around 70 g/cm$^2$ as measured by liquid chromatography.

Example 33

Example 32 is repeated except the nitroglycerine containing paste is replaced with 48.49% pseudoephedrine in petroleum jelly as the reservoir.

13

The article so formed exhibits uniform and prolonged in-vitro pseudoephedrine release for a period of over 30 hours with a release rate around 2 g/cm$^2 \cdot$ hr as measured by UV spectrophotometric method.

Example 34

This example illustrates the utility of the present polymer system in seed coating.

Thus, about 20 grams each of corn and soybean seeds are dip coated with the solution prepared in Example 20. After draining the excess coating solution, the coated seeds are blended with bentonite to eliminate tackiness. The coated seeds are dried under room conditions and then subjected to standard germination tests.

The results of the germination tests indicate that the seed coating slightly delays the germination time but does not affect the total percentage of seed germination.

Example 35

This example further illustrates use of the present polymer system in seed coating.

25 parts of a 25% aqueous solution of polyacrylic acid having an average molecular weight of 50,000 is blended with 5 parts of the nonyl phenoxy polyethoxy ethanol according to Example 12 and 3.33 parts of isopropanol to form a viscous solution having a 1 to 1 polycarboxylic acid to ethoxylated surfactant ratio. 5.66 parts of the above solution are then mixed with 20 parts of a 10% polyvinyl alcohol aqueous solution to form a stable blend having a solids content of 15.6%.

The blend so formed is used to coat about 4 grams each of corn and soyabean seeds according to the procedures described in Example 34. After air drying, a clear and elastic coating is observed.

Example 36

This example further illustrates the use of the present polymer system in seed coating and its effect on seed germination.

2.22 parts of a 45% aqueous solution of polyacrylic acid having an average molecular weight of 5,000 is blended with 1 part of the nonylphenoxy polyethoxy ethanol used in Example 35 and 0.61 parts of water to form a viscous solution of solid content 52.2% having a 1 to 1 polycarboxylic acid to ethoxylated surfactant ratio.

About 1 kg each of corn, soyabean and sorghum seeds are spray-coated on a laboratory granulator using the coating solution prepared here. Talc and saw dust are added to eliminate tackiness during the coating process. The coated seeds are dried under room conditions and subjected to standard germination tests.

The results of the germination test are given below. They show that the germination of the coated seeds is almost the same as of the uncoated samples. No adverse effects are observed.

| Coated seed | Storage period (days) | Germination (%) |
|---|---|---|
| Corn | 0 | 91 (93*) |
|  | 30 | 79 (94*) |
| Soybean | 0 | 98 (98*) |
|  | 60 | 96 |
| Sorghum | 0 | 83 (93*) |
|  | 60 | 87 |

*) uncoated sample

Example 37

This example illustrates the preparative versatility of the polymer systems of the present invention.

Thus, the polymer solutions described in Examples 20 and 29 hereinabove are blended together to form a 1 to 1 mixture. Upon drying overnight at room temperature conditions, a good elastic, clear film product is obtained.

Example 38

This example further illustrates the unexpected properties of the instant polymeric systems, particularly in comparison to the systems according to US—A—3.387.061 to Smith et al.

A polymer representative according to Smith et al except for the low molecular weight of the polymeric ether compound is prepared whereby 25 parts of poly(ethylene glycol) having about 10 ethylene oxide units and an average molecular weight of 400 are blended with 125 parts of 20%, by weight, aqueous solution of polyacrylic acid having an average molecular weight of 250,000. As noted by Smith, the blend is devoid of precipitation. A similar result is observed when poly(ethylene glycol) having about 5 ethylene oxide units and an average molecular weight of 200 is utilized in the blend. These solutions are then air dried at room temperature. The resulting solids exhibit multiple glass transitions upon being measured by differential scanning calorimetry, indicating a mixture morphology.

In contrast, an immediate increase in viscosity followed by precipitation is observed when a polymeric system according to the present invention is prepared utilizing 25 parts of nonylphenoxy polyethoxy ethanol having about 5 ethylene oxide units, an HLB value of 10 and an average molecular weight of 400 blended with 125 parts of 20%, by weight, aqueous solution of polyacrylic acid having an average molecular weight of 250,000. Comparable results are achieved when the nonionic surfactant is nonylphenoxy polyethoxy ethanol having 9 to 10 ethylene oxide units, an HLB value of 13.4 and an average molecular weight of 642. After air drying at room temperature, the resulting precipitates exhibit single glass transition values as determined by differential scanning calorimetry, similar to the pattern exhibited by homopolymers. The benefits derived from the presence of the nonionic surfactant of the instant invention are thus apparent.

Example 39

This example further illustrates the distinction in utility between the general systems described in Example 37.

27.5 parts of nonylphenoxy polyethoxy ethanol having 9 to 10 ethylene oxide units, an HLB value of 13.4 and an average molecular weight of 642 are blended with 100 parts of 27.5%, by weight, aqueous solution of polyacrylic acid having an average molecular weight of 250,000, 37.2 parts of deionized water and 8.6 parts of isopropanol to form a solution with a solids content of 31.7% and a Brookfield viscosity of 8 Pa · s at 25°C.

The resulting solution is knife coated with an 0,275 mm (11 mil) wet laydown onto the surface of a 0,075 mm (3 mil) buisiness grade paper (Hyolite Sub 40, trademark) to form a paper supported membrane. After drying in a hot air oven at 80°C, the paper supported membrane is formed into a sealed pouch containing citrus essential oil in the same manner as described in Example 31. A second sealed pouch is also prepared based on a similar paper supported membrane except that the coating solution utilizing nonylphenoxy polyethoxy ethanol having about 5 ethylene oxide units, an HLB value of 10 and an average molecular weight of 440 as the surfactant component (as Example 37). These sealed pouches exhibit uniform and prolonged releases of citrus essential oil with an average $t_{50}$ of 10 days and an average total effective release period of over two months.

In contrast, when the above mentioned process is repeated utilizing low molecular weight poly(ethylene glycol) of either 5 or 10 ethylene oxide units in place of the nonylphenoxy polyethoxy ethanol, the resulting sealed pouch exhibits an almost negligible release (less than 5%) of citrus essential oil over a period of two months.

It can therefore be seen from Examples 37 and 38 that the instant systems containing the prescribed nonionic surfactants are quite distinct from systems containing a low molecular weight equivalent of the Smith et al polymeric ether compounds in flexibility of use and particularly in the ability to provide prolonged and controlled release of volatile active ingredients.

Summarizing, it is seen that this invention provides unique polymeric systems capable of a wide range of applications.

**Claims**

1. A coated absorbant substrate, coated on one or both surfaces thereof with a polymeric system comprising an association product of a polymeric carboxylic acid having free carboxyl groups on at least 10% of the monomer units thereof, and an ethoxylated nonionic surfactant having at least 2 ethylene oxide units and a maximum molecular weight of about 4000, said absorbant substrate being impregnated with a volatile and/or water-soluble active agent, which are released at a controlled and uniform rate.

2. The substrate according to claim 1, wherein said acid and said surfactant of the association product are present in a weight ratio of from 1:20 to 20:1.

3. The substrate according to claim 1, wherein said acid of the association product is selected from the group consisting of homopolymers of unsaturated carboxylic acids; copolymers of acrylic or methacrylic acid with at least one polymerizable vinyl compound; copolymers of acrylic or methacrylic acid with at least one polymerizable vinylidene compound; hydrolyzed interpolymers of alpha, beta-ethylenically unsaturated dicarboxylic acid anhydrides with terminally unsaturated mono-olefins, cyclic terpenes, vinyl compounds, vinylidene compounds, acrylic acid and esters thereof and methacrylic acid and esters thereof; the carboxyalkylated derivatives of polyhydroxy polymers; hydrolyzed polymers containing acrylamide or acrylonitrile groups; polycarboxylic acid ethers based on pentanoic acid residues; carboxy polymethylene hydrocolloid; and the partial esters and amides of said acids.

4. The substrate according to claim 3, wherein said acid of the association product is selected from the group consisting of polyacrylic acid, polyacrylic acid crosslinked with approximately 1% of polyallyl sucrose, polymethacrylic acid, polymaleic acid, polyitaconic acid, polyhydroxybenzoic acid, polygalacturonic acid, polyglutamic acid, polyglycollic acid, polylactic acid, ethylene/acrylic acid copolymer, ethylene/methacrylic acid copolymer, methylmethacrylate/methacrylic acid copolymer, methyl acrylate/methyl methacrylate/methacrylic acid terpolymer, ethyl acrylate/t-butyl acrylamid/acrylic acid terpolymer, methyl vinyl ether/maleic acid copolymer, vinyl acetate/crotonic acid copolymer, butadiene/maleic acid copolymer, polymaleic anhydride, acrylonitrile/maleic anhydride copolymer, butadiene/maleic anhydride copolymer, ethylene/maleic anhydride copolymer, 1-hexene/maleic anhydride copolymer, 1-octadecene/maleic anhydride copolymer, methyl vinyl ether/maleic anhydride copolymer, n-octadecyl vinyl ether/maleic anhydride copolymer, styrene/maleic anhydride copolymer, vinyl acetate/maleic anhydride copolymer, cellulose acetate phthalate, carboxymethyl cellulose, and carboxyl modified polyacrylamide.

5. The substrate according to claim 4, wherein said acid of the association product is polyacrylic acid, an ethylene-maleic acid copolymer, styrene-maleic anhydride copolymer, 1-hexene-maleic anhydride copolymer, ethylacrylate/t-butyl acrylamide/acrylic acid terpolymer, polymethacrylic acid or cellulose acetate phthalate.

6. The substrate according to claim 1, wherein said ethoxylated nonionic surfactant of the association product is selected from the group consisting of ethoxylated alkylphenols, ethoxylated mono- and polyhydroxy aliphatic alcohols, ethoxylated fatty amines, ethoxylated fatty acid amides, ethoxylated fatty acid ethanolamides, ethoxylated fatty acids, ethoxylated fatty acid esters, ethoxylated sorbitan fatty acid esters, ethoxylated sorbitol esters, ethoxylated vegetable oils, ethoxylated lanolin derivatives, ethoxylated sugar derivatives, ethoxylated naphthalene derivatives, ethoxylated mercaptans, ethoxylated polypropylene glycols, ethoxylated fatty glycerides, ethoxylated fatty glycol esters and ethoxylated sucroglycerides.

7. The substrate according to claim 6, wherein said ethoxylated nonionic surfactant of the association product is selected from the group consisting of octylphenoxy polyethoxy ethanols, nonylphenoxy polyethoxy ethanols, ethoxylated sorbitan monolaurates, ethoxylated sorbitan monopalmitates, ethoxylated sorbitan monostearates, ethoxylated sorbitan monooleates, ethoxylated sorbitan tristearates, ethoxylated sorbitan trioleates, ethoxylated oleyl amides, ethoxylated tallow amides, ethoxylated glycol laurates, ethoxylated glycol stearates, ethoxylated glycol oleates, ethoxylated stearic acid, ethoxylated oleic acid, ethoxylated resin fatty acids, ethoxylated lauryl ethers, ethoxylated cetyl ethers, ethoxylated stearyl ethers, ethoxylated oleyl ethers, ethoxylated tridecyl ethers, ethoxylated polydimethylsiloxanes, ethoxylated polypropylene glycols, ethoxylated polyurethanes, and ethoxylated perfluoroalkyl polyurethanes.

8. The substrate according to claim 1, wherein said ethoxylated nonionic surfactant of the association product has a hydrophile-lipophile balance value of from about 2 to 40.

9. The absorbent coated substrate according to claim 1, which is impregnated with a volatile active agent, said polymeric system is coated on one surface thereof and a barrier backing impermeable to said volatile active agent is affixed to the uncoated surface of said substrate, said volatile active agent being released at a controlled and uniform rate.

10. A laminate structure comprising two substrates according to claim 1, each coated on one surface thereof and impregnated with a volatile active agent, said laminate being formed by placing the uncoated surface of each substrate in face-to-face contact and adhering same, said volatile active agent being released at a controlled and uniform rate.

11. A shaped article prepared from a polymeric system comprising an association product of a polymeric carboxylic acid having free carboxyl groups on at least 10% of the monomer units thereof, and an ethoxylated nonionic surfactant having at least 2 ethylene oxide units and a maximum molecular weight of about 4000, which contains a volatile and/or water-soluble active agent therein, said active agent being released at a controlled and uniform rate.

12. A solid, water-soluble active agent coated with a polymeric system comprising an association product of a polymeric carboxylic acid having free carboxyl groups on at least 10% of the monomer units thereof, and an ethoxylated nonionic surfactant having at least 2 ethylene oxide units and a maximum molecular weight of about 4000, said water-soluble active agent being released at a controlled and uniform rate.

13. A sealed pouch prepared from the substrate according to claim 1.

14. The pouch of claim 13, wherein said active agents are present on an absorbent substrate.

15. Seed coated with a polymeric system comprising an association product of a polymeric carboxylic acid having free carboxyl groups on at least 10% of the monomer units thereof, and an ethoxylated nonionic surfactant having at least 2 ethylene oxide units and a maximum molecular weight of about 4000.

**Patentansprüche**

1. Ein umhülltes absorptionsfähiges Substrat, das an einer oder beiden Oberflächen mit einem polymeren System umhüllt ist, welches aus einem Assoziationsprodukt einer polymeren Carbonsäure, die an zumindest 10% ihrer Monomer-Einheiten freie Carboxylgruppen aufweist, und einem ethoxylierten nichtionischen oberflächenaktiven Mittel, das zumindest 2 Ethylenoxid-Einheiten aufweist und ein maximales Molekulargewicht von etwa 4000 hat, besteht, wobei dieses absorptionsfähige Substrat mit

**0 075 540**

einem flüchtigen und/oder wasserlöslichen aktiven Stoff imprägniert ist, der in kontrollierter und gleichmässiger Geschwindigkeit freigesetzt wird.

2. Das Substrat gemäss Anspruch 1, worin besagte Säure und besagtes oberflächenaktives Mittel des Assoziationsprodukts in einem Gewichtsverhältnis von 1:20 bis 20:1 vorhanden sind.

3. Das Substrat gemäss Anspruch 1, worin besagte Säure des Assoziationsprodukts ausgewählt ist aus der Gruppe bestehend aus Homopolymeren von ungesättigten Carbonsäuren; Copolymeren von Acryl- oder Methacrylsäure mit zumindest einer polymerisierbaren Vinylverbindung; Copolymeren von Acryl- oder Methacrylsäure mit zumindest einer polymerisierbaren Vinylidenverbindung; hydrolysierten Interpolymeren von alpha, beta-ethylenisch ungesättigten Dicarbonsäureanhydriden mit . terminal ungesättigten Monoolefinen, cyclischen Terpenen, Vinylverbindungen, Vinylidenverbindungen, Acrylsäure und Estern davon und Methacrylsäure und Estern davon; den carboxyalkylierten Derivaten von Polyhydroxypolymeren; hydrolysierten Polymeren, die Acrylamid- oder Acrylnitrilgruppen enthalten; Polycarbonsäureethern, die auf Pentansäure-Resten basieren; Carboxypolymethylen-Hydrokolloiden; und den Partialestern und -amiden dieser Säuren.

4. Das Substrat gemäss Anspruch 3, worin besagte Säure des Assoziationsproduktes ausgewählt ist aus der Gruppe bestehend aus Polyacrylsäure, Polyacrylsäure, die mit ungefähr 1% Polyallylsucrose vernetzt ist, Polymethacrylsäure, Polymaleinsäure, Polyitaconsäure, Polyhydroxybenzoesäure, Polygalacturonsäure, Polyglutaminsäure, Polyglycolsäure, Polymilchsäure, Ethylen/Acrylsäure - Copolymer, Ethylen/Methacrylsäure - Copolymer, Methylmethacrylat/Methacrylsäure - Copolymer, Methylacrylat/Methylmethacrylat/Methacrylsäure - Terpolymer, Ethylacrylat/t - Butylacrylamid/Acrylsäure - Terpolymer, Methylvinylether/Maleinsäure - Copolymer, Vinylacetat/Crotonsäure - Copolymer, Butadien/Maleinsäure - Copolymer, Polymaleinsäureanhydrid, Acrylnitril/Maleinsäureanhydrid - Copolymer, Butadien/Maleinsäureanhydrid - Copolymer, Ethylen/Maleinsäureanhydrid - Copolymer, 1 - Hexen/Maleinsäureanhydrid - Copolymer, 1 - Octadecen/Maleinsäureanhydrid - Copolymer, Methylvinylether/Maleinsäureanhydrid - Copolymer, n - Octadecylvinylether/Maleinsäureanhydrid - Copolymer, Styrol/Maleinsäureanhydrid - Copolymer, Vinylacetat/Maleinsäureanhydrid - Copolymer, Celluloseacetatphthalat, Carboxymethylcellulose und Carboxyl-modifiziertes Polyacrylamid.

5. Das Substrat gemäss Anspruch 4, worin besagte Säure des Assoziationsproduktes Polyacrylsäure ist, ein Ethylen/Maleinsäure - Copolymer, Styrol/Maleinsäureanhydrid - Copolymer, 1 - Hexen/Maleinsäureanhydrid - Copolymer, Ethylacrylat/t - Butylacrylamid/Acrylsäure - Terpolymer, Polymethacrylsäure oder Celluloseacetatphthalat.

6. Das Substrat gemäss Anspruch 1, worin besagtes oberflächenaktives Mittel des Assoziationsproduktes ausgewählt ist aus der Gruppe bestehend aus ethoxylierten Alkylphenolen, ethoxylierten Mono- und Polyhydroxy-aliphatischen Alkoholen, ethoxylierten Fettaminen, ethoxylierten Fettsäureamiden, ethoxylierten Fettsäureethanolamiden, ethoxylierten Fettsäuren, ethoxylierten Fettsäureestern, ethoxylierten Sorbitanfettsäureestern, ethoxylierten Sorbitestern, ethoxylierten pflanzlichen Oelen, ethoxylierten Lanolinderivaten, ethoxylierten Zuckerderivaten, ethoxylierten Naphthalinderivaten, ethoxylierten Mercaptanen, ethoxylierten Polypropylenglycolen, ethoxylierten Fettsäureglyceriden, ethoxylierten Fettsäureglycolestern und ethoxylierten Sucroglyceriden.

7. Das Substrat gemäss Anspruch 6, worin besagtes oberflächenaktives Mittel des Assoziationsproduktes ausgewählt ist aus der Gruppe bestehend aus Octylphenoxypolyethoxyethanolen, Nonylphenoxypolyethoxyethanolen, ethoxylierten Sorbitanmonolauraten, ethoxylierten Sorbitanmonopalmitaten, ethoxylierten Sorbitanmonostearaten, ethoxylierten Sorbitanmonooleaten, ethoxylierten Sorbitantristearaten, ethoxylierten Sorbitantrioleaten, ethoxylierten Oleylamiden, ethoxylierten Talgamiden, ethoxylierten Glycollauraten, ethoxylierten Glycolstearaten, ethoxylierten Glycololeaten, ethoxylierter Stearinsäure, ethoxylierter Oelsäure, ethoxylierten Harz-Fettsäuren, ethoxylierten Laurylethern, ethoxylierten Cetylethern, ethoxylierten Stearylethern, ethoxylierten Oleylethern, ethoxylierten Tridecylethern, ethoxylierten Polydimethylsiloxanen, ethoxylierten Polypropylenglycolen, ethoxylierten Polyurethanen und ethoxylierten Perfluoralkylpolyurethanen.

8. Das Substrat gemäss Anspruch 1, worin besagtes ethoxyliertes nichtionisches oberflächenaktives Mittel des Assoziationsproduktes einen Hydrophilie-Lipophilie-Balancewert von etwa 2 bis 40 hat.

9. Das umhüllte absorptionsfähige Substrat gemäss Anspruch 1, das mit einem flüchtigen aktiven Stoff imprägniert ist, auf dessen eine Oberfläche besagtes polymere System als Umhüllung aufgebracht ist und auf dessen nicht umhüllte Oberfläche eine rückwärtige Barriere aufgebracht ist, die für besagten flüchtigen aktiven Stoff undurchlässig ist, wobei dieser flüchtige aktive Stoff in kontrollierter und gleichmässiger Geschwindigkeit freigesetzt wird.

10. Eine Schichtstruktur enthaltend zwei Substrate gemäss Anspruch 1, jedes an einer seiner Oberflächen umhüllt und mit einem flüchtigen aktiven Stoff imprägniert, wobei die Schichtstruktur dadurch gebildet wird, dass die nicht umhüllten Oberflächen jedes Substrates in Kopf-Kopf-Kontakt gebracht und verklebt werden, wobei der flüchtige aktive Stoff in kontrollierter und gleichmässiger Geschwindigkeit freigesetzt wird.

11. Ein geformter Artikel, hergestellt aus einem polymeren System, welches aus einem Assoziationsprodukt einer polymeren Carbonsäure, die an zumindest 10% ihrer Monomer-Einheiten freie Carboxylgruppen aufweist, und einem ethoxylierten nichtionischen oberflächenaktiven Mittel, das zumindest 2 Ethylenoxid-Einheiten aufweist und ein maximales Molekulargewicht von etwa 4000 hat,

17

**0 075 540**

besteht, der einen flüchtigen und/oder wasserlöslichen aktiven Stoff enthält, wobei dieser aktive Stoff in kontrollierter und gleichmässiger Geschwindigkeit freigesetzt wird.

12. Ein fester wasserlöslicher aktiver Stoff, der mit einem polymeren System umhüllt ist, welches aus einem Assoziationsprodukt einer polymeren Carbonsäure, die an zumindest 10% ihrer Monomer-Einheiten freie Carboxylgruppen aufweist und einem ethoxylierten nichtionischen oberflächenaktiven Mittel, das zumindest 2 Ethylenoxid-Einheiten aufweist und ein maximales Molekulargewicht von etwa 4000 hat, besteht, wobei dieser wasserlösliche aktive Stoff in kontrollierter und gleichmässiger Geschwindigkeit freigesetzt wird.

13. Eine versiegelte Tasche, hergestellt aus dem Substrat gemäss Anspruch 1.

14. Die Tasche gemäss Anspruch 13, worin besagte aktive Stoffe auf einem absorbierenden Substrat enthalten sind.

15. Saatgut, das mit einem polymeren System umhüllt ist, welches aus einem Assoziationsprodukt einer polymeren Carbonsäure, die an zumindest 10% ihrer Monomer-Einheiten freie Carboxylgruppen aufweist, und einem ethoxylierten nichtionischen oberflächenaktiven Mittel, das zumindest 2 Ethylenoxid-Einheiten aufweist und ein maximales Molekulargewicht von etwa 4000 hat, besteht.

**Revendications**

1. Un support absorbant revêtu, qui est revêtu sur une de ses faces ou sur les deux par un système polymère comprenant un produit d'association d'un acide carboxylique polymère contenant des groupes carboxyle libres sur au moins 10% de ses motifs monomères, et un agent tensio-actif non-ionique éthoxylé contenant au moins deux motifs d'oxyde d'éthylène et présentant un poids moléculaire maximum d'environ 4 000, ce support absorbant étant imprégné d'un agent actif volatil et/ou soluble dans l'eau qui est libéré à une vitesse contrôlée et uniforme.

2. Le support selon la revendication 1, dans lequel l'acide et l'agent tensio-actif du produit d'association sont présents dans des proportions relatives en poids de 1:20 à 20:1.

3. Le support selon la revendication 1, dans lequel l'acide du produit d'association est choisi dans le groupe consistant en les homopolymères d'acides carboxyliques insaturés; les copolymères de l'acide acrylique ou méthacrylique avec au moins un composé vinylique polymérisable; les copolymères de l'acide acrylique ou méthacrylique avec au moins un composé vinylidénique polymérisable; les interpolymères hydrolysés d'anhydrides d'acides dicarboxyliques à insaturation alpha, bêta-éthylénique avec des monooléfines à insaturation terminale, des terpènes cycliques, des composés vinyliques, des composés vinylidéniques, l'acide acrylique et ses esters et l'acide méthacrylique et ses esters; les dérivés carboxyalkylés de polymères polyhydroxylés; les polymères hydrolysés contenant des groupes acrylamide ou acrylonitrile; les éthers d'acides polycarboxyliques à base de radicaux d'acide pentanoïque; les hydrocolloïdes de carboxypolyméthylène; et les esters et amides partiels de ces acides.

4. Le support selon la revendication 3, dans lequel l'acide du produit d'association est choisi dans le groupe consistant en l'acide polyacrylique, l'acide polyacrylique réticulé par environ 1% de polyallyl-saccharose, l'acide polyméthacrylique, l'acide polymaléique, l'acide polyitaconique, l'acide polyhydroxy-benzoïque, l'acide polygalacturonique, l'acide polyglutamique, l'acide polyglycolique, l'acide polylactique, un copolymère éthylène/acide acrylique, un copolymère éthylène/acide méthacrylique, un copolymère méthacrylate de méthyle/acide méthacrylique, un copolymère ternaire acrylate de méthyle/méthacrylate de méthyle/acide méthacrylique, un copolymère ternaire acrylate d'éthyle/tert - butylacrylamide/acide acrylique, un copolymère éther méthylvinylique/acide maléique, un copolymère acétate de vinyle/acide crotonique, un copolymère butadiène/acide maléique, l'anhydride polymaléique, un copolymère acrylonitrile/anhydride maléique, un copolymère butadiène/anhydride maléique, un copolymère éthylène/anhydride maléique, un copolymère 1-hexène/anhydride maléique, un copolymère 1-octadécène/anhydride maléique, un copolymère éther méthylvinylique/anhydride maléique, un copolymère éther n - octadécylvinylique/anhydride maléique, un copolymère styrène/anhydride maléique, un copolymère acétate de vinyle/anhydride maléique, de l'acétatephtalate de cellulose, la carboxyméthylcellulose et un polyacrylamide à modification carboxyle.

5. Le support selon la revendication 4, dans lequel l'acide du produit d'association est l'acide polyacrylique, un copolymère éthylène/acide maléique, un copolymère styrène/anhydride maléique, un copolymère 1-hexène/anhydride maléique, un copolymère ternaire acrylate d'éthyle/tert - butylacrylamide/acide acrylique, l'acide polyméthacrylique ou l'acétate-phtalate de cellulose.

6. Le support selon la revendication 1, dans lequel l'agent tensio-actif non-ionique éthoxylé du produit d'association est choisi dans le groupe consistant en les alkylphénols éthoxylés, les alcools aliphatiques mono- et poly-hydroxylés éthoxylés, les amines grasses éthoxylées, les amides d'acides gras éthoxylés, les éthanolamides d'acides gras éthoxylés, les acides gras éthoxylés, les esters d'acides gras éthoxylés, les esters d'acides gras et de sorbitanne éthoxylés, les esters de sorbitol éthoxylés, les huiles végétales éthoxylées, les dérivés éthoxylés de la lanoline, les dérivés éthoxylés de sucres, les dérivés éthoxylés de naphtalène, les mercaptans éthoxylés, les polypropylènes-glycols éthoxylés, les glycérides gras éthoxylés, les esters de glycols gras éthoxylés et les saccharoglycérides éthoxylés.

7. Le support selon la revendication 6, dans lequel l'agent tensio-actif non-ionique éthoxylé du produit d'association est choisi dans le groupe consistant en les octylphénoxy- polyéthoxy-éthanols, les

18

nonylphénoxy - polyéthoxy - éthanols, les monolaurates de sorbitanne éthoxylés, les monopalmitates de sorbitanne éthoxylés, les monostéarates de sorbitanne éthoxylés, les monooléates de sorbitanne éthoxylés, les tristéarates de sorbitanne éthoxylés, les trioléates de sorbitanne éthoxylés, les oléylamides éthoxylés, les amides de suif éthoxylés, les laurates de glycols éthoxylés, les stéarates de glycols éthoxylés, les oléates de glycols éthoxylés, l'acide stéarique éthoxylé, l'acide oléique éthoxylé, les acides gras résiniques éthoxylés, les éthers lauryliques éthoxylés, les éthers cétyliques éthoxylés, les éthers stéaryliques éthoxylés, les éthers oléyliques éthoxylés, les esters tridécyliques éthoxylés, les polydiméthyl-siloxannes éthoxylés, les polypropylène-glycols éthoxylés, les polyuréthannes éthoxylés et les perfluor-alkylpolyuréthannes éthoxylés.

8. Le support selon la revendication 1, dans lequel l'agent tensio-actif non-ioniques éthoxylé du produit d'association présente une valeur de l'équilibre hydrophile-lipophile d'environ 2 à 40.

9. Le support absorbant revêtu selon la revendication 1, qui est imprégné d'un agent actif volatil, le système polymère étant appliqué en revêtement sur une face dudit support et un dossier de barrière imperméable à l'agent actif volatil étant fixé sur la face non revêtue dudit support, cet agent actif volatil étant libéré à une vitesse contrôlée et uniforme.

10. Une structure stratifiée comprenant deux supports selon la revendication 1, revêtus chacun sur une de leurs faces et imprégnés d'un agent actif volatil, ce stratifié étant formé en plaçant la face non revêtue de chaque support en contact face à face avec l'autre face non revêtu et en faisant adhérer ces faces, l'agent actif volatil étant libéré à une vitesse contrôlée et uniforme.

11. Un article formé préparé à partir d'un système polymère comprenant un produit d'association d'un acide carboxylique polymère portant des groupes carboxyle libre sur au moins 10% de ses motifs monomères, et un agent tensio-actif non-ionique éthoxylé contenant au moins deux motifs d'oxyde d'éthylène et présentant un poids moléculaire maximum d'environ 4 000, qui contient à l'intérieur un agent actif volatil et/ou soluble dans l'eau, cet agent actif étant libéré à une vitesse contrôlée et uniforme.

12. Un agent actif solide, soluble dans l'eau, revêtu d'un système polymère comprenant un produit d'association d'un acide carboxylique polymère portant des groupes carboxyle libres sur au moins 10% de ses motifs monomères, et un agent tensio-actif non-ionique éthoxylé contenant au moins deux motifs d'oxyde d'éthylène et présentant un poids moléculaire maximum d'environ 4 000, cet agent actif soluble dans l'eau étant libéré à une vitesse contrôlée et uniforme.

13. Une poche fermée préparée à partir du support selon la revendication 1.

14. La poche selon la revendication 13, dans laquelle les agents actifs sont présents sur un support absorbant.

15. Semence revêtue d'un système polymère comprenant un produit d'association d'un acide carboxylique polymère contenant des groupes carboxyle libres sur au moins 10% de ses motifs monomères, et un agent tensio-actif non-ionique éthoxylé contenant au moins deux motifs d'oxyde d'éthylène et présentant un poids moléculaire maximum d'environ 4 000.

FIG. 1

FIG. 2

FIG. 3

FIG. 4